(19) Europäisches Patentamt
European Patent Office
Office européen des brevets

(11) **EP 3 308 763 B1**

(12) **EUROPEAN PATENT SPECIFICATION**

(45) Date of publication and mention
of the grant of the patent:
**07.06.2023  Bulletin 2023/23**

(21) Application number: **16807868.1**

(22) Date of filing: **13.06.2016**

(51) International Patent Classification (IPC):
*A61H 31/00* (2006.01)    *A61N 1/39* (2006.01)
*A61B 5/372* (2021.01)    *A61B 5/00* (2006.01)

(52) Cooperative Patent Classification (CPC):
**A61B 5/0245; A61B 5/026; A61B 5/14551;**
**A61B 5/282; A61B 5/316; A61B 5/369;**
**A61B 5/374; A61B 5/4836; A61B 5/6814;**
**A61B 5/6824; A61B 5/702; A61B 5/7282;**
**A61H 31/006; A61N 1/39044;** A61B 5/361;  (Cont.)

(86) International application number:
**PCT/KR2016/006229**

(87) International publication number:
**WO 2016/200228 (15.12.2016 Gazette 2016/50)**

(54) **AUTOMATIC CARDIOPULMONARY RESUSCITATION DEVICE**

AUTOMATISCHE HERZ-LUNGEN-REANIMATIONSVORRICHTUNG

DISPOSITIF DE RÉANIMATION CARDIOPULMONAIRE AUTOMATIQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **12.06.2015  KR 20150083283**
**25.08.2015  KR 20150119266**

(43) Date of publication of application:
**18.04.2018  Bulletin 2018/16**

(73) Proprietors:
• **Mediana Co., Ltd.**
**Wonju-si, Gangwon-do 26365 (KR)**
• **SNU R & DB Foundation**
**Seoul 08826 (KR)**

(72) Inventors:
• **LEE, Sungho**
**VVonju-si**
**Gangwon-do 26421 (KR)**
• **LIM, Hacgyu**
**Wonju-si**
**Gangwon-do 26438 (KR)**
• **LEE, Heetack**
**Wonju-si**
**Gangwon-do 26388 (KR)**

• **KANG, Dongwon**
**Wonju-si**
**Gangwon-do 26482 (KR)**
• **KIM, Eongsok**
**Wonju-si**
**Gangwon-do 26399 (KR)**
• **Shin, Sang Do**
**Seoul 04558 (KR)**
• **Kim, Hee Chan**
**Seoul 04427 (KR)**
• **Hong, Ki Jeong**
**Seoul 07993 (KR)**
• **Kim, Hee Jin**
**Seoul 02840 (KR)**

(74) Representative: **Gulde & Partner**
**Patent- und Rechtsanwaltskanzlei mbB**
**Wallstraße 58/59**
**10179 Berlin (DE)**

(56) References cited:
KR-A- 20110 014 186    KR-A- 20110 116 519
KR-B1- 100 282 733    KR-B1- 101 206 581
US-A1- 2014 324 763    US-A1- 2014 324 763
US-A1- 2014 358 047    US-A1- 2015 105 636

- **DEEPAK K TEMPE ET AL: "Is there any alternative to the Bispectral Index Monitor?", BRITISH JOURNAL OF ANAESTHESIA, vol. 92, no. 1, 1 January 2004 (2004-01-01), XP055542824, DOI: 10.1093/bja/aeh002**
- **Murali Chakravarthy ET AL: "Bispectral Index Is an Indicator of Adequate Cerebral Perfusion During Cardiopulmonary Resuscitation", , 10 September 2003 (2003-09-10), XP055542800, DOI: 10.1016/S1053-0770(03)00160-6 Retrieved from the Internet: URL:https://www.sciencedirect.com/science/article/pii/S1053077003001605/pdfft?md5=5e129beaf443dd57a441aec4ea6b4987&pid=1-s2.0-S1053077003001605-main.pdf [retrieved on 2019-01-16]**
- **N. AZIM ET AL: "The use of bispectral index during a cardiopulmonary arrest: a potential predictor of cerebral perfusion : Bispectral index during cardiopulmonary arrest", ANAESTHESIA., vol. 59, no. 6, 1 June 2004 (2004-06-01), pages 610-612, XP055542790, GB ISSN: 0003-2409, DOI: 10.1111/j.1365-2044.2004.03736.x**
- **Stryker Corporation: "LUCAS 2 CHEST COMPRESSION SYSTEM", , 31 December 2014 (2014-12-31), XP055918134, Retrieved from the Internet: URL:https://www.lucas-cpr.com/files/3650333_LUCAS%202%20Quick%20Reference%20Card%20I ntl%20Eng%203303572_D_LR.PDF [retrieved on 2022-05-04]**
- **RAMPIL I J: "A Primer for EEG Signal Processing in Anesthesia", ANESTHESIOLOGY, AMERICAN SOCIETY ANESTHESIOLOGISTS, US, vol. 89, no. 4, 1 October 1998 (1998-10-01), pages 980-1002, XP008083318, ISSN: 0003-3022, DOI: 10.1097/00000542-199810000-00023**
- **Riegger Christian: "Messung des Bispektralindex und der Suppression Ratio als frühzeitiger Prädiktor für das neurologische Outcome nach kardiopulmonaler Reanimation", , 1 January 2009 (2009-01-01), XP055974711, Retrieved from the Internet: URL:https://oparu.uni-ulm.de/xmlui/bitstream/handle/123456789/2017/vts_7128_10007.pdf?isAllowed=y&sequence=1 [retrieved on 2022-10-25]**

(52) Cooperative Patent Classification (CPC): (Cont.)
A61B 2505/01; A61H 31/008; A61H 2205/084;
A61H 2230/105; A61H 2230/208

**Description**

[Technical Field]

The present invention relates to a cardiopulmonary resuscitation device that measures electrocardiogram and oxygen saturation during a cardiopulmonary resuscitation procedure, detects brain activity in real time which is a degree of blood circulation to the brain by measuring electroencephalogram, is equipped with a defibrillation function, uses a brain activation index as a CPR signal indicating a degree of cardiopulmonary resuscitation, and is capable of bio signal remote monitoring in a pre-hospital step.

[Background Art]

Cardiopulmonary resuscitation (CPR) is an emergency treatment method that helps breathing by artificially circulating blood when a heart attack (cardiac arrest) occurs, and when an acute cardiac arrest that suddenly stops the heart of a patient occurs, cardiopulmonary resuscitation must be performed as a first aid. That is, simultaneously performing artificial respiration and cardiac compression (heart massage) is called cardiopulmonary resuscitation.

The general cardiopulmonary resuscitation recovers around 20% of the normal blood flow, but when the automated external defibrillator (AED) is used, a 100% recovery is possible, and the AED can increase the survival rate of a cardiac arrest patient up to 70%.

The automated external defibrillator (AED) is configured as a device for performing cardiopulmonary resuscitation, and generally AED is configured to automatically shock a patient by checking the heart condition of patient.

When a heart attack occurs, blood circulation to the whole body is interrupted so that if appropriate measures are not taken right away, a patient may die or a serious brain damage may occur. In particular, the brain begins to be irreversibly injured even when blood supply is interrupted for 4 to 5 minutes, if blood is not supplied to the brain for 6 minutes or more, the functions of the brain and all organs of a patient are stopped, and the patient may die.

The conventional auto cardiopulmonary resuscitation device detects the chest compression rate and uses it as a CPR signal which indicates the degree of cardiopulmonary resuscitation. However, detecting brain activity can be a more accurate detection method for the degree of cardiopulmonary resuscitation.

Therefore, an auto cardiopulmonary resuscitation device is desired which measures electrocardiogram, oxygen saturation, and also electroencephalogram, detects brain activity which is the degree of blood circulation in the brain by using the measured electroencephalogram, remotely sends the detected data to a medical professional, and performs electric shock using a built-in automated external defibrillator in dangerous situations, while performing cardiopulmonary resuscitation.

In particular, it is necessary to use a cardiopulmonary resuscitation device as fast as possible under emergency situations, and it is highly likely that the device is used in a narrow space such as an ambulance, and therefore, an auto cardiopulmonary resuscitation device is desired which is equipped with devices for detection, calculation, and transmission of electrocardiogram, oxygen saturation, and brain activity, and an automatic defibrillator.

Korean Laid-Open Patent Publication No. 10-2011-0014186 relates to a device for cardiopulmonary resuscitation (CPR) including a chest compression unit, and this chest compression unit includes a plunger located in housing and an electric motor control unit, and the plunger is operated in a reciprocating-motion manner by a reversible electric motor or by a linear flow electric motor, and the electric motor control unit calculates the pressure applied by the plunger and controls the displacement of the plunger based on this pressure.

In Korean Laid-Open Patent Publication No. 10-2011-0014186, the electric motor control unit can receive oxygen saturation, ECG data, EEG data, etc., and these data are used for electric motor control. It is stated that it is possible to control the management of defibrillation pulse and to interlock it.

However, Korean Laid-Open Patent Publication No. 10-2011-0014186 does not disclose any details regarding calculation processes used for oxygen saturation, ECG data, EEG data, etc., parameters that are extracted by the calculation processes, and methods of using extracted data to control an electric motor or automated external defibrillator by, and in addition, this cardiopulmonary resuscitation device does not include a device for detecting and analyzing oxygen saturation, ECG, and EEG.

Laid-Open Patent Publication No. US 2014/0324763 A1 discloses systems and methods to predict the chances of neurologically intact survival while performing CPR. According to one aspect, a method for predicting the likelihood of survival of a particular individual with favorable neurological function during a CPR procedure includes obtaining an electroencephalogram (EEG) signal of the particular individual during the CPR procedure.

In general, in an emergency situation where a cardiopulmonary resuscitation device is used, people panic, and in such a situation, it is not easy to find measurement devices for oxygen saturation, ECG, and EEG, and take measurements by attaching each electrode of ECG, EEG, etc. to appropriate positions for patients.

[DISCLOSURE]

[Technical Problem]

The problem that the present invention intends to solve is to provide an auto cardiopulmonary resuscitation device which measures electrocardiogram and oxygen saturation during a cardiopulmonary resuscitation procedure, detects brain activity in real time which is a degree of blood circulation to the brain by measuring electroencephalogram, is equipped with a defibrillation function, uses a brain activation index as a CPR signal indicating a degree of cardiopulmonary resuscitation, and is capable of bio (*in vivo*) signal remote monitoring in a pre-hospital step.

The problem that the present invention intends to solve is to provide an auto cardiopulmonary resuscitation device which uses a brain activation index as a CPR signal indicating the degree of cardiopulmonary resuscitation and displays the brain activation index as a CPR signal (CPR feedback information) by waveform or numerical values on a monitor unit of the auto cardiopulmonary resuscitation device.

The problem that the present invention intends to solve is to provide an auto cardiopulmonary resuscitation device which uses a brain activation index as a CPR signal indicating the degree of cardiopulmonary resuscitation, and also the brain activation index uses a BSR, beta ratio, and SynchFastSlow (SFS).

Another problem that the present invention intends to solve is to provide an auto cardiopulmonary resuscitation device that is equipped with devices which detect electrocardiogram, oxygen saturation, and brain activity, and send data detected by these devices remotely to a medical professional, and is equipped with an automated external defibrillator configured to be able to perform electric shock using a built-in automated external defibrillator in a dangerous situation.

Another problem that the present invention intends to solve is to provide an auto cardiopulmonary resuscitation which calculates and processes oxygen saturation, ECG data, and EEG data to extract parameters and controls an operational motor or an automated defibrillator by using the extracted parameters.

[Technical Solution]

In order to solve the above problem, the cardiopulmonary resuscitation device according to the present invention includes an electroencephalogram detection unit configured to detect an electroencephalogram signal in the frontal lobe; a calculation processing unit configured to detect a brain activation index from the electroencephalogram signal received from the electroencephalogram detection unit in which the cardiopulmonary resuscitation device uses a brain activation index as a CPR signal indicating a degree of cardiopulmonary resuscitation, wherein the calculation processing unit calculates one or more of a burst suppression ratio (BSR), a beta ratio, and a relative synchrony of fast and slow wave (SynchFastSlow, SFS) as the brain activation index; a main body portion equipped with a chest compression unit configured to pressure a chest of a patient; and a holder configured to support the main body portion and be able to attach or detach the main body portion, wherein the holder further comprises: a main body holding unit which is U-shaped and bound to an edge of the main body portion; a holder leg portion which is connected to the main body holding unit to support the main body portion; and a back support portion, wherein one end thereof is connected to the holder leg portion as a means of supporting a back of a patient, wherein the back support portion is composed of two boards, and wherein a distance between the two boards is adjusted to correspond to the width of the body of a patient.

In embodiments, the calculation processing unit removes a noise in a electroencephalogram signal received by the electroencephalogram detection unit, passes the electroencephalogram signal in which the noise is removed through a 1 Hz low pass filter, performs correction by excluding the signal that passed through the 1Hz low pass filter in the electroencephalogram signal in which the noise has been removed, takes a first derivative of the corrected electroencephalogram signal, detects a section in which an amplitude is 5 $\mu$V or less for 0.5 sec or more as a suppression (flat brain wave), and thereby detects BSR.

The calculation processing unit removes a noise in an electroencephalogram signal received by the electroencephalogram detection unit and calculates a beta ratio from the electroencephalogram signal in which the noise has been removed according to:

$$\text{Beta Ratio} = \log [\text{spectral power (30-47Hz)} / \text{spectral power (11-20Hz)}]$$

(Note that spectral power (30-47Hz) is a power spectrum at a frequency of 30 to 47 Hz in electroencephalogram, and spectral power (11-20Hz) is a power spectrum at a frequency of 11 to 20Hz).

The calculation processing unit removes a noise in an electroencephalogram signal received by the electroencephalogram detection unit and may calculate SynchFastSlow (SFS) from the electroencephalogram signal in which the noise has been removed according to:

SynchFastSlow=log [bispectral power (0.5-47.0Hz)/ bispectral power (40.0-47.0Hz)]

(Note that bispectral power (0.5-47.0Hz) is a bispectrum calculated in a range of 0.5 Hz to 47.0Hz, and bispectral power (40.0-47.0Hz) is a bispectrum calculated in a range of 40.0Hz to 47.0Hz).

The calculation processing unit assigns a score according to pre-stored distribution values for each of BSR, beta ratio, and SynchFastSlow (SFS) and adds up assigned scores of BSR, beta ratio, and SynchFastSlow (SFS) to calculate a brain activity.

The cardiopulmonary resuscitation device includes a main body portion equipped with a chest compression unit configured to pressure a chest of a patient; and a holder configured to support the main body portion wherein the main body portion can be attached thereto or detached therefrom.

The holder further includes a main body holding unit which is U-shaped and bound to an edge of the main body portion; a holder leg portion which is connected to the main body holding unit to support the main body portion; and a back support portion wherein one end thereof is connected to the holder leg portion as a means of supporting a back of a patient.

The cardiopulmonary resuscitation device further includes an angle adjusting unit equipped with a hinge between the holder leg portion and back portion in which the angle adjusting unit can be fixed by a stopper.

The cardiopulmonary resuscitation device further includes a wrist fastening unit configured to fasten and secure a wrist of a patient to left and right sides of the main body holding unit.

The wrist fastening unit includes any one of an electrocardiogram sensor and oxygen saturation sensor to detect an electrocardiogram signal or oxygen saturation signal to send to the calculation processing unit.

The back support portion includes an electrocardiogram sensor to detect an electrocardiogram signal to send to the calculation processing unit.

The electroencephalogram detection unit includes two or four electroencephalogram signal electrodes, and the electroencephalogram detection unit may be headband-formed.

The cardiopulmonary resuscitation device further includes a monitor unit configured to output a brain activation index.

In addition, a method for detecting brain activity using the cardiopulmonary resuscitation device that uses a brain activation index as a CPR signal indicating a degree of cardiopulmonary resuscitation which is carried out by the calculation processing unit and the transceiver unit of said device and which is not forming part of the present invention includes: a noise removing step wherein the calculation processing unit removes a noise in an electroencephalogram signal received from the electroencephalogram detection unit; a suppression detection step wherein the calculation processing unit passes the electroencephalogram signal in which the noise is removed through a 1 Hz low pass filter, performs correction by excluding the signal that passed through the 1Hz low pass filter in the electroencephalogram signal in which the noise has been removed, takes a first derivative of the corrected electroencephalogram signal, and detects a section in which an amplitude is equal to or below a suppression reference amplitude for a time equal to or longer than a suppression reference time as a suppression (flat brain wave); a BSR calculation step wherein the calculation processing unit detects a burst suppression ratio (BSR) using a time interval of suppression in the suppression detection step in the corrected electroencephalogram signal in the suppression detection step.

Here, the suppression reference amplitude is 5 μV, and the suppression reference time is 0.5 sec. The suppression reference may be 5 μV or less and the suppression reference time may be 0.5 sec or more.

The method further includes an FFT calculation step and a beta ratio calculation step. The FFT calculation step is that the calculation processing unit performs FFT to the electroencephalogram signal in which the noise is removed in the noise removing step to calculate a spectral power. The beta ratio calculation step is that the calculation processing unit uses the spectral power calculated in the FFT calculation step to calculate a beta ratio according to:

$$\text{Beta Ratio} = \log [\text{spectral power (30-47 Hz)}/ \text{spectral power (11-20 Hz)}]$$

(Note that spectral power (30- 47 Hz) is a power spectrum at a frequency of 30 to 47 Hz in electroencephalogram, and spectral power (11-20 Hz) is a power spectrum at a frequency of 11 to 20 Hz).

The method further includes a bispectrum calculation step and a SynchFastSlow (SFS) calculation step. The bispectrum calculation step is that the calculation processing unit uses a spectral power calculated in the FFT calculation step to calculate a bispectrum in a range of 0.5 Hz to 47.0 Hz and a bispectrum in a range of 40.0 Hz to 47.0 Hz. The SynchFastSlow (SFS) calculation step is that the calculation processing unit uses the bispectral power (0.5-47.0 Hz) in a range of 0.5 Hz to 47.0 Hz and the bispectral power (40.0-47.0 Hz) in a range of 40.0 Hz to 47.0 Hz to calculate SynchFastSlow (SFS) according to:

$$\text{SynchFastSlow}=\log [\text{bispectral power (0.5-47.0 Hz)}/ \text{bispectral power (40.0-47.0 Hz)}].$$

The method further includes a brain activity calculation step wherein the calculation processing unit assigns each score for each value of BSR, beta ratio, and SynchFastSlow (SFS) and adds up each assigned score to calculate a brain activity.

The noise removing further includes a band pass filtering step, an independent component analysis step, a template matching step, and an electrooculogram (EOG) signal removing step. The band pass filtering step is that the calculation processing unit passes an electroencephalogram signal received from the electroencephalogram detection unit through a band pass filter having a pass band of 0.1 Hz to 50 Hz. The independent component analysis step is that the calculation processing unit separates independent components (source signal) in the electroencephalogram signal passed through the band pass filter according to the independent component analysis (ICA). The template matching step is that the calculation processing unit calculates correlation coefficients between a template indicating pre-stored EOG and the independent components, and determines the independent component that a coefficient value of the correlation coefficient exceeds pre-stored reference range of coefficient values as an electrooculogram (EOG) signal. The electrooculogram (EOG) signal removing step is that the calculation processing unit removes the electrooculogram (EOG) signal detected by the template matching step in the independent components (source signal) separated by the independent component analysis step.

The method further includes a motion artifact removing step wherein the calculation processing unit determines as a motion artifact and remove when variance of electroencephalogram signals in which an electrooculogram (EOG) signal is removed in the electrooculogram (EOG) signal removing step, exceeds a pre-stored reference value.

[Advantageous Effects]

The auto cardiopulmonary resuscitation device according to the present invention measures electrocardiogram and oxygen saturation during a cardiopulmonary resuscitation procedure, detects brain activity in real time which is a degree of blood circulation to the brain by measuring electroencephalogram, is equipped with a defibrillation function, and uses a brain activation index as a CPR signal indicating a degree of cardiopulmonary resuscitation, and therefore, it is possible to provide appropriate treatments to patients in less time so that the rate of resuscitation of patients can be increased

In addition, the present invention uses a brain activation index as a CPR signal indicating the degree of cardiopulmonary resuscitation and displays the brain activation index as a CPR signal (CPR feedback information) by waveform and numerical values on a monitor unit of the auto cardiopulmonary resuscitation device so that even when a user is a beginner, the user can recognize the status of a patient more easily.

In addition, the present invention uses a brain activation index as a CPR signal indicating the degree of cardiopulmonary resuscitation, and also the brain activation index uses a BSR, beta ratio, and SynchFastSlow (SFS) to increase accuracy.

In addition, the auto cardiopulmonary resuscitation device according to the present invention is equipped with devices which detect electrocardiogram, oxygen saturation, and brain activity, and send data detected by these devices remotely to a medical professional, and is equipped with an automated external defibrillator configured to be able to perform electric shock using a built-in automated external defibrillator in a dangerous situation. Therefore, the device is equipped with devices for detecting various bio signals and also composed as a compact instrument equipped with a defibrillator equipped, and since the device can be used easily and rapidly without panic at moments when patients need to be treated fast, not only the rate of resuscitation of a patient can be increased, but also the it is convenient to use the device even in a narrow space like an ambulance.

In addition, the auto cardiopulmonary resuscitation device according to the present invention extracts the parameter by calculating and processing oxygen saturation, ECG data, and EEG data, and controls an operational motor or an automated external defibrillator by using the extracted parameters so that beginners can easily use the device.

[Description of Drawings]

FIG. 1 is an example of the auto cardiopulmonary resuscitation device according to the present invention.

FIG. 2 is a disassembly perspective view in which a main body portion and a holder of the auto cardiopulmonary resuscitation device of FIG. 1 are disassembled.

FIG. 3 is a usage status diagram showing an example of the usage status of the auto cardiopulmonary resuscitation device according to the present invention.

FIG. 4 is a block diagram for schematically explaining the configuration of the auto cardiopulmonary resuscitation device 10 according to the present invention.

FIG. 5 is a schematic diagram for schematically explaining the signal transfer process of the auto cardiopulmonary resuscitation device and the main server 400 according to the present invention.

FIG. 6 shows an example of the headband-type electroencephalogram detection unit according to the present invention.

FIG. 7 is a flowchart for schematically explaining a process of detecting a brain activity at the calculation processing unit of FIG. 4.

[BEST MODE]

Hereinafter, the auto cardiopulmonary resuscitation device according to the present invention will be described in detail with reference to the accompanying drawings.

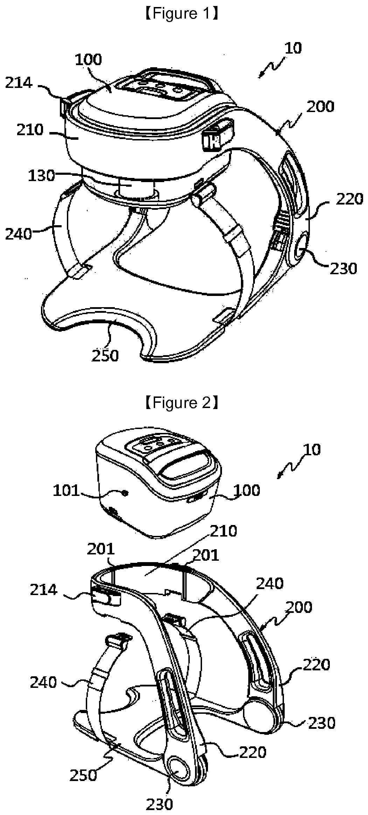

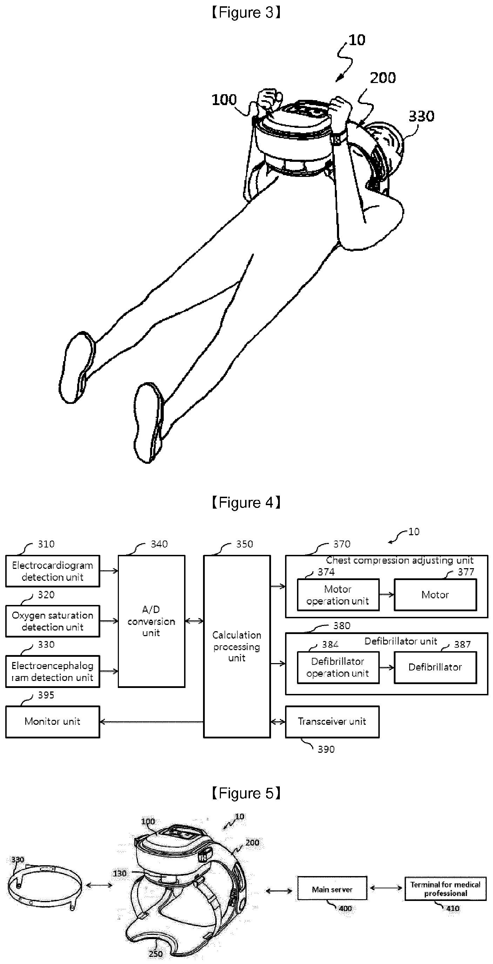

FIG. 1 is an example of the auto cardiopulmonary resuscitation device according to the present invention, FIG. 2 is a disassembly perspective view in which a main body portion and a holder of the auto cardiopulmonary resuscitation device of FIG. 1 are disassembled, and FIG. 3 is a usage status diagram showing an example of the usage status of the auto cardiopulmonary resuscitation device according to the present invention.

The auto cardiopulmonary resuscitation device 10 includes a main body portion 100 and a holder 200.

The main body portion 100 is constituted so as to be detachable from the holder 200 and is equipped with a chest compression adjustment unit 370 so as to operate a chest compression unit 130 to perform chest compression during cardiopulmonary resuscitation. One end of the chest compression unit 130 is fitted with a chest compression disc or chest compression suction cup.

In addition, the main body portion 100 receives the extracted electrocardiogram (ECG) signals, oxygen saturation signals, electroencephalogram (EEG, brain wave) signals detected from an electrocardiogram detection unit 310, an oxygen saturation detection unit 320, and an electroencephalogram detection unit 330. Further, the main body portion 100 detects parameters from each extracted signal and sends to a main server 410, and also detects brain activity from brain wave. In addition, the main body portion 100 may further include a defibrillator unit 380. According to circumstances, the main body portion 100 can control a motor operation unit 374 of the chest compression adjusting unit 370 or control the defibrillator unit 380 based on the brain activity.

Here, an electrocardiogram sensor section (not shown by a diagram) of the electrocardiogram detection unit 310, an oxygen saturation sensor section (not shown by a diagram) of the oxygen saturation detection unit 320, and an electrocardiogram sensor unit (not shown by a diagram) of an electroencephalogram sensor unit 330 (not shown by a diagram) can be constituted so that they can be attached separately to a patient. Alternatively, according to circumstances, an electrocardiogram sensor unit (not shown by a diagram) and an oxygen saturation sensor unit (not shown by a diagram) can be mounted on the holder 200. The electroencephalogram detection unit 330 can be constituted in the form of a headband attached to the head and can be constituted so as to detect electroencephalogram signals in the frontal lobe.

On a side of the main body portion 100, a convexity 101 is equipped for coupling with a groove 201 of the main body holding unit 210.

The holder 200 includes a main body holding unit 210, a holder leg portion 220, a back support portion 250, an auxiliary supporting portion 240, and a wrist fastening unit 214.

The main body holding unit 210 is constituted to be in a U-shaped form as a means for connecting with a side of the main body portion 100 and is connected to a holder leg portion 220. he main body holding unit 210 is equipped with a groove 201 on the inside thereof and the groove 201 is combined with the convexity 101 of the main body portion 100 to fix the main body holding unit 210 and the main body portion 100. As shown in FIGs. 2 and 3, since the main body holding unit 210 is constituted to be in a U-shaped, it has an opening. When CPR device is installed at a patient, the opening is positioned around the neck of the patient.

The support leg portion 220 maintains the main body portion 100 supported by the main body holding unit 210 and is a means of support so that the main body portion 100 and the back support portion 250 are separated by a fixed distance. The holder leg portion 220 includes two holder legs, and the neck and head of a patient are inserted (positioned) between the two holder legs. As shown in FIG. 3, the holder legs are connected at each end of the main body holding unit 210.

Between the holder leg portion 220 and the back support portion 250, an angle adjustment unit 230 is provided and is constituted so that the distance between a bottom (base) of the main body portion 100 (i.e. the base of the chest compression disc or chest compression suction cup) and the back support portion 250, that is, the distance between the bottom of the main body portion 100 (i.e. the base of the chest compression disc or chest compression suction cup) and the chest of a patient, can be adjusted. An angle adjusting unit 230 is equipped with a hinge and also with a stopper, and is configured so as to adjust the angle between the main body holding unit 210 and the back support portion 250 for fixing. By doing so, the device can be used by adjusting the angle adjusting unit 230 regardless of whether the patient is an adult, a child, an obese person, or a lean person.

The back support portion 250 is a board to be placed on the back of a patient. In FIG. 1, the back support portion 250 is constituted with one board, but according to the invention, the one board is replaced by two separate boards.

By adjusting the space between the two boards, a width can be formed so that the device can used regardless of adults, children, obese people, or lean people. According to circumstances, an electrocardiogram sensor unit (not shown by a diagram) can be located in the back supporting portion.

An auxiliary support portion 240 is a chest compression belt provided to be connected between the back support portion 250 and the main body portion 100 so as to wrap and compress the chest of a patient and is provided on each of the right and left sides. One end of the auxiliary support portion 240 is fixed to the side of the back support portion 250, and the other end of the auxiliary support portion 240 is connected to a ring fixed to the lower side of the main body portion 100 to be fixed thereto.

A wrist fastening unit 214 is a pair of wrist binding bands for binding and fixing the wrist of a patient. The wrist fastening unit 214 is provided on both the left and right sides of the main body holding unit 210 and is configured to fix the wrist of a patient during cardiopulmonary resuscitation, so as not to be disturbed by movements of hands or arms during cardiopulmonary resuscitation. According to circumstances, the wrist fastening unit can be equipped with an electrocardiogram sensor unit (not shown by a diagram) or oxygen saturation sensor unit (not shown by a diagram).

FIG. 4 is a block diagram for schematically explaining the configuration of the auto cardiopulmonary resuscitation device 10 according to the present invention and includes an electrocardiogram detection unit 310, an oxygen saturation detection unit 320, an electroencephalogram detection unit 330, an A/D conversion unit 340, a calculation processing unit 350, a chest compression adjusting unit 370, a defibrillator unit 380, a transceiver unit 390, and a monitor unit 395.

The electrocardiogram detection unit 310 includes an electrocardiogram sensor unit (not shown by a diagram) for detecting an electrocardiogram signal, and an electrocardiogram signal preprocessing unit (not shown by a diagram) for amplifying the electrocardiogram signal detected from the electrocardiogram sensor unit and removing noise. The electrocardiogram signal detected by the electrocardiogram detection unit 310 is transmitted to the calculation processing unit 350 via the A/D conversion unit 340. An electrocardiogram sensor unit (not shown by a diagram) can be mounted on the back support portion 250 and can be attached to the wrist fastening unit 214, and according to circumstances, it can be separated from an auto cardiopulmonary resuscitation device 10, and equipped with another electrocardiogram sensor unit (not shown by a diagram) placed on the body.

The oxygen saturation detection unit 320 includes an oxygen saturation sensor unit (not shown by a diagram) for detecting an oxygen saturation signal, and an oxygen saturation signal preprocessing unit (not shown by a diagram) for amplifying an oxygen saturation signal detected by the oxygen saturation sensor unit and removing noise thereof. The oxygen saturation signal detected by the oxygen saturation detection unit 320 is transmitted to the calculation processing unit 350 via the A/D conversion unit 340. An oxygen saturation sensor unit (not shown by a diagram) is constituted with a light emitting unit and a light receiving unit, and in the light emitting unit, an infrared wavelength light emitting diode (LED) and red wavelength LED are used. The oxygen saturation sensor unit can be mounted on the back support portion 250 and can be mounted on the wrist fastening unit 214. According to circumstances, the oxygen saturation sensor unit can be separated from the auto cardiopulmonary resuscitation device 10 to be equipped to a separate oxygen saturation sensor unit (not shown by a diagram) attached on the patient body (for example, a finger, etc.).

The electroencephalogram detection unit 330 includes an electroencephalogram sensor unit (not shown by a diagram) for detecting an electroencephalogram signal, and an electroencephalogram signal preprocessing unit (not shown) for amplifying the electroencephalogram signal detected from the electroencephalogram sensor unit and removing noise, and the electroencephalogram signal detected by the electroencephalogram detection unit 330 is transmitted to the calculation processing unit 350 via the A/D conversion unit. The electroencephalogram detection unit 330 is constituted in a headband type so that a brain electrode can be attached to the frontal lobe, and the electroencephalogram signal detected by the electroencephalogram detection unit 330 can be transmitted to the calculation processing unit 350 by wire or wireless.

In the A/D conversion unit 340, electrocardiogram (ECG) signals, oxygen saturation signals, and electroencephalogram (EEG) signals received from the electrocardiogram detection unit 310, the oxygen saturation detection unit 320, and the electroencephalogram detection unit 330 are converted into digital signals and sent to the calculation processing unit 350.

The calculation processing unit 350 receives electrocardiogram (ECG) signals, oxygen saturation signals, and electroencephalogram (EEG) signals, detects each parameter by calculation processing. The calculation processing unit 350 compares each parameter with each reference value pre-stored in a memory unit (not shown by a diagram) to determine whether it is risk or not. Further, the calculation processing unit 350 sends each parameter obtained calculated from the received electrocardiogram (ECG) signals, oxygen saturation signals, and electroencephalogram (EEG) signals to a main server (central management center server) 400 or a terminal for medical professionals 410.

The calculation processing unit 350 detects heart rates, ventricular fibrillation, etc. from electrocardiogram (ECG) signals and detects arterial blood oxygen saturation, venous blood oxygen saturation, an oxygen dissociation (oxygen dissociation curve), etc. from oxygen saturation signals. This is widely known, and a detailed description thereof will be omitted herein.

In addition, the calculation processing unit 350 detects brain activity from electroencephalogram (EEG) signals and determines the degree of cardiopulmonary resuscitation by using the detected brain activity.

According to circumstances, the calculation processing unit 350 determines the degree of cardiopulmonary resuscitation by using the detected brain activity or by using brain activity, heart rates, ventricular fibrillation, arterial blood oxygen saturation, etc., and a motor control signal can be generated which changes chest compression rates based on the degree of cardiopulmonary resuscitation and send it to the chest compression adjusting unit 370. In addition, the calculation processing unit 350 determines the degree of cardiopulmonary resuscitation by using the detected brain activity or by using brain activity, heart rates, ventricular fibrillation, arterial blood oxygen saturation, etc. and when the degree of cardiopulmonary resuscitation is out of a fixed reference range, a defibrillator control signal can be generated and sent to the defibrillator unit 380.

The chest compression adjusting unit 370 is a means for controlling the chest compression unit 130 so as to perform chest compression at a predetermined rate and includes a motor operation unit 374 and a motor 377, and is configured to operate a chest compression unit 130 by operating the motor 377 according to the motor control signal received from the calculation processing unit 350.

The motor operation unit 374 operates the motor 377 according the motor control signal received from the calculation processing unit 350.

The motor 377 is operated by the motor operation unit 374, and the motor 377 is linked with a plunger of the chest compression unit 130.

The chest compression unit 130 includes a plunger, and the plunger is operated in the manner of reciprocating motions by the motor 377, that is, a reversible electric motor via a mechanical means for converting rotational motion into linear motion.

A defibrillator unit 380 is a means for operating a defibrillator and includes a defibrillator operation unit 384 and a defibrillator 387.

The defibrillator operation unit 384 operates the defibrillator 387 in response to defibrillator control signals of the calculation processing unit 350 or by a user turning on the key of the defibrillator.

The defibrillator 387 is equipped with a left-side electrode pad and a right-side electrode pad and applies electric shock to the heart of a patient via the left-side electrode pad and right-side electrode pad according to the defibrillator operation unit 384.

The transceiver unit 390 transmits the electrocardiogram, electroencephalogram, oxygen saturation, brain activity, heart rates, ventricular fibrillation, arterial blood oxygen saturation, etc., received from the calculation processing unit 350 to the main server 400.

The monitor unit 395 displays electrocardiogram, electroencephalogram, oxygen saturation, brain activity, heart rates, ventricular fibrillation, arterial blood oxygen saturation, etc. received from the calculation processing unit 350. In addition, it displays emergency situations when the degree of cardiopulmonary resuscitation falls outside a fixed reference range. In particular, the monitor unit 395 outputs brain activity or brain activation index (brain activation indicator) as a CPR signal indicating the degree of cardiopulmonary resuscitation.

According to circumstances, the present invention can further include a respirator (not shown by a diagram), and can further include an additional breathing capacity measuring sensor portion (not shown by a diagram).

FIG. 5 is a schematic diagram for schematically explaining the signal transfer process of the auto cardiopulmonary resuscitation device and the main server 400 according to the present invention.

The auto cardiopulmonary resuscitation device 10 receives the electroencephalogram from the electroencephalogram detection unit 330, receives electrocardiogram and oxygen saturation from the electrocardiogram detection unit 310 and the oxygen saturation detection unit 320 mounted on the holder 200, and then calculates brain activity, heart rate, ventricular fibrillation, arterial blood oxygen saturation, etc. The auto cardiopulmonary resuscitation device 10 transmits data such as electrocardiogram, electroencephalogram, oxygen saturation, brain activity, heart rate, ventricular fibrillation, arterial blood oxygen saturation, etc. to the main server 400.

The main server 400 sends the received data to the terminal for medical professionals 410 and transmits the diagnosis of the medical professionals received from the terminal for medical professional 410 to the auto cardiopulmonary resuscitation device 10.

In the electroencephalogram detection unit 330, electrode attachment sites use the internationally unified 10-20 electrode placement method (10-20 system) and can attach multiple electrodes to the frontal lobe part. For example, signal electrodes for electroencephalogram detection can be attached to a frontal pole (Fp) of the frontal lobe, that is, Fp1 and Fp2.

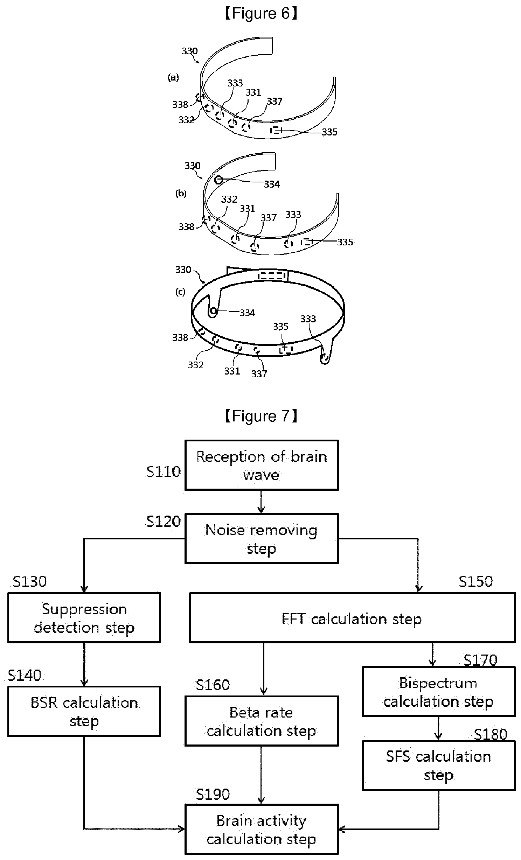

FIG. 6 shows an example of the headband-type electroencephalogram detection unit according to the present invention.

FIG. 6(a) shows that the electroencephalogram detection unit 330 is equipped with signal electrodes 331, 332, 337, 338 at Fp1, Fp2, Fp7, Fp8 of the frontal lobe, respectively, and a grounding electrode 333 is provided in the center thereof. According to circumstances, the signal electrode can be attached only Fp1 and Fp2.

FIG. 6(b) shows that the electroencephalogram detection unit 330 is equipped with signal electrodes 331, 332, 337, 338 at Fp1, Fp2, Fp7, Fp8 of the frontal lobe, respectively, and is equipped with a grounding electrode 333 in one side of the right and left sides, and accordance to circumstances, a reference electrode 334 is provided on one side of the right and left sides. According to circumstances, the signal electrode can be attached only Fp1 and Fp2.

FIG. 6(c) shows that the electroencephalogram detection unit 330 is equipped with signal electrodes 331, 332, 337, 338 at Fp1, Fp2, Fp7, Fp8 of the frontal lobe, respectively, and at the skull behind the ears on one side of the right and left sides, a grounding electrode 333 is provided, and according to circumstances, a reference electrode 334 is provided at the skull behind the ears on the other one side the right and left sides. In addition, according to circumstances, the signal electrode can be attached only Fp1 and Fp2.

The headband-type electroencephalogram detection unit of FIG. 6(a) to 6(c) is possible to further include the electroencephalogram detection circuit module 335 that is positioned on one side of the headband. The electroencephalogram detection circuit module 335 includes an electroencephalogram signal preprocessing unit (not shown by a diagram) and an electroencephalogram signal transmitter (not shown by a diagram).

Although FIG. 6 describes an electroencephalogram detection unit of the headband type, this is not intended to limit the present invention, and according to circumstances, the electroencephalogram detection unit can be constituted as a disposable adhesive patch type so that it can be used by attaching to the frontal lobe of a patient.

FIG. 7 is a flowchart for schematically explaining a process of detecting a brain activity at the calculation processing unit of FIG. 4.

In the noise removing step S120, the calculation processing unit 350 removes a noise in the electroencephalogram signal (brain wave signal) received from the A/D conversion unit 340.

In this noise removing step, first, the calculation processing unit 350 passes electroencephalogram signals through a bandpass filter (not shown by a diagram) that has a passband of 0.1 Hz to 50 Hz. The bandpass filter can be constituted as a tertiary Butterworth filter.

Independent components (that is, source signals) are separated according to the independent component analysis (ICA) from the electroencephalogram signal that has passed through the band pass filter. In general, when an electroencephalogram (brain wave) signal is detected at the frontal lobe, an electrooculogram (EOG) signal is added in this electroencephalogram signal. Since it is difficult to determine which signal is the electrooculogram (EOG) signal among the independent components (that is, source signals) separated according to the independent component analysis method (ICA), the correlation coefficient between a pre-stored EOG template and the independent components (that is, source signals) is calculated. When the coefficient value of the correlation coefficient exceeds a pre-stored reference range of the coefficient values, it determines the signal as the electrooculogram (EOG) signal and removes the detected electrooculogram (EOG) signal among the independent components. Calculating the correlation with the independent components and the EOG template, and detecting the electrooculogram (EOG) signal among independent components (that is, source signals) based on the coefficient value may say to be a template matching.

When using four electroencephalogram electrodes (Fp1, Fp2, Fp7, and Fp8), processing is carried out by applying the independent component analysis (ICA) method for the electroencephalogram signal that has passed through the above band pass filter to detect a mixed matrix and four independent components (i.e., source signals). Further, the channel of EOG signals among the four independent components (i.e., 4 source signal channels) is detected by pattern matching between EOG signal features of the EOG template (i.e., reference EOG or EOG signal features with square waves) and the four independent components.

In addition, motion artifacts which are noises by the motion of patient, are removed in the electroencephalogram signals that are removed the electrooculogram (EOG) signal. Variance, that is, the difference between the current signal value and previous signal value, is calculated in real time in the electroencephalogram signal in which the electrooculogram (EOG) signal has been removed, and if the variance is exceeds a fixed reference value, it determines the signal as a motion artifact and remove it. That is, a section in which variance sharply increases in the electroencephalogram signal in which the electrooculogram (EOG) signal has been removed is detected and removed.

As a suppression detection step S130, an electroencephalogram signal (raw-wave signal) in which noise has been removed in the noise removing step S120 is passed through a 1 Hz low pass filter so as to consider baseline wandering, and suppression detection (flat brain wave detection) is carried out.

That is, it is carried out correction by deducting the signal which has passed through the 1 Hz low pass filter from the electroencephalogram signal (raw-signal) in which noise has been removed, and is performed first derivative about the corrected electroencephalogram signal, and detects a section in which the amplitude continuously is 5 μV or

less for 0.5 sec or more, as a suppression (flat brain wave). According to circumstances, instead of a 1 Hz low pass filter, it can be taken a moving average of the base line of the above electroencephalogram.

In general, in the criteria of burst suppression, flat brain wave is a section that the amplitude maintains at a 5 $\mu$V or less for 0.5 sec or more, but when these criteria are suddenly applied to electroencephalogram raw-wave signals (i.e., original electroencephalogram signal), suppression cannot be accurately obtained by base line drift. Therefore, the correction as described above is performed. Peak detection is performed based on the first derivative of this waveform to calculate the amplitude of the appearance, and calculate burst suppression ratio (BSR, ratio of burst to flat brain wave ratio) by applying the criteria (i.e., definitions) of the burst suppression. In general, the moving average is the simplest low pass filter, which becomes a low cut filter when moving average is subtracted.

In a BSR calculation step 140, suppression (flat brain wave) detected in the suppression detection step S130 is used in the electroencephalogram signal corrected in the suppression detection step S130 and is used to detect burst flat brain wave ratio (BSR).

The burst flat brain wave ratio (BSR) is calculated as the ratio of a burst (brain wave with high potential at high frequency) to suppression (brain wave with low potential, flat brain wave) detected at the suppression detection step S130. In this case, it is determined as a burst when a voltage having a pre-stored burst reference amplitude or more lasts for a burst reference time or longer, and it is determined as suppression when the voltage having a pre-stored suppression reference amplitude below lasts for a suppression reference time or longer, and then the ratio of time interval of the burst and the time interval of the suppression can be obtained. According to circumstances, the total time interval of bursts and the total time interval of suppression in a given time interval can be obtained. According to circumstances, BSR can be a value in which the total time interval of suppression is divided by the total time interval of burst, and expressed as a percentage.

Alternatively, the burst flat brain wave ratio (BSR) is detected by the ratio of the suppressions (flat brain waves) detected in the suppression detection step S130. In this case, it is determined as a suppression when the voltage having a suppression reference amplitude or more lasts for a suppression reference time or longer, and the ratio occupied by the suppression time during the measurement time of the brain wave can be obtained.

According to circumstances, BSR can be value in which the total time of suppression during a given time is divided by the brain wave measurement time and is expressed as a percentage.

In addition, according to circumstances, in BSR calculation step 140, QUAZI (also called QUAZI suppression rate) which indicates the ratio of pre bursts can be further obtained. QUAZI can use the data obtained by performing the power spectrum analysis for the electroencephalogram signals. Burst and suppression (flat brain wave) are detected according to a predetermined standard (that is, the stored QUAZI discrimination standard) in the waveform obtained by removing component with a frequency of 1 Hz or less from the electroencephalogram signal (raw-wave signal) to obtain the ratio of the time. Here, the predetermined standard (QUAZI criterion), that is, the discrimination standard for burst and suppression (flat brain wave), is lower than the BSR discrimination criteria. That is, QUAZI is used to quantify a little lower level, that is, a pre-burst state, than when burst and suppression appears in BSR. QUAZI (inhibition rate) can predict the present inhibition rate in the currently unstable voltage of a baseline. In general, QUAZI is an index (indicator) used when the baseline sway is large in the flat part of burst suppression, and mainly used for detecting a deep sleep state.

In an FFT calculation step S150, FFT is performed on the electroencephalogram signal (raw-wave signal) in which noise has been removed in the noise removing step S120 to obtain spectral power. According to circumstances, the Blackman window function can be used before performing FFT in the FFT calculation step S150.

In a beta ratio calculation step S160, using the spectral power in the FFT calculation step S150, the beta ratio is obtained. The beta ratio (Beta Ratio) is obtained by

$$\text{Beta Ratio} = \log\left[\text{spectral power (30-47 Hz)}/\text{spectral power (11-20 Hz)}\right]$$

Here, the spectral power (30-47 Hz) is the power spectrum of the gamma band in the brain wave, and the spectral power (11-20 Hz) is the power spectrum of the beta band.

According to circumstances, the beta ratio may be calculated as the log ratio of the bispectrum peak sum in the 11 to 20 Hz region and the bispectrum peak sum in the 30 to 47 Hz region.

In a bispectrum calculation step S170, bispectrum is calculated in the range of 0.5 Hz to 47.0 Hz, and in addition, bispectrum is calculated in the range of 40.0 Hz to 47.0 Hz. Here, calculating the bispectrum in the range of 0.5 Hz to 47.0 Hz means that bispectrum is calculated at f1 = 0.5 Hz, f2 = 47.0 Hz, and f1 + f2 = 47.5 Hz.

In additional explanation, bispectral analysis investigates a nonlinear interaction (phase coupling) of a waveform having two different frequencies. As a simple example of phase coupling, a function that returns the square of an input as an output can be mentioned. The input is the sum of two cosine functions of frequencies that are f1 and f2, and the respective phases are q1 and q2, and the output function at this time is as follows.

INPUT:

$$x(t) = \cos(f1t+q1) + \cos(f2t+q2)$$

OUTPUT:

$$y(t) = 1 + \cos[(f1+f2)t+(q1+q2)] + \cos[(f1-f2)t+(q1-q2)] + 1/2\cos(2f1t+2q1) +$$

$$1/2\cos(2f2t+2q2)$$

In this case, the frequency components of f1 + f2, f1 - f2, 2f1, and 2f2 are dependent on the components of the frequencies of f1 and f2. The components created by these nonlinear equations (calculations other than addition/subtraction of inputs) are called intermodulation products (IMPs). As IMPs, the phase depends on the phase of original components as shown by the above equations. These IMP components are expressed as phase-couple. Meanwhile, components that are not IMPs are called fundamental. Bispectral analysis is an interpretation method for examining the existence and extent of the phase-coupled components of the signal component. If it is a linear combination of waves having other frequencies and existing waves, it can be decomposed into individual frequency components by the power spectral analysis. However, in the case of nonlinear coupling, it cannot be interpreted by power spectral analysis. Considering two different frequencies f1 and f2 and the added frequency f1 + f2, bispectral analysis sets the f1 wave and the f2 wave to X(f1) and X(f2), respectively, the phase thereof can be set as fit + q1 and f2t + q2, and the phase of the wave of f1 + f2 can be (f1+f2)t+q3. Wave is represented by a two-dimensional parameter generally called size and phase which are shown as a complex number. Here, if the conjugate complex number of X (f 1 + f 2) is X * (f 1 + f 2), the phase of X * (f 1 + f 2) is the inversion of the phase of X (f 1 + f 2). That is, - {(f1 + f2) t + q3}. Considering the triple product of the products of X (f 1), X (f 2), and X * (f 1 + f 2), the phase of this triple product is (f 1 t + q 1) + (f 2 t + q 2) - {(f 1 + f 2) t + q 3} = q 1 + q 2 - q 3. If there is no phase coupling between q 1, q 2, and q 3, the phase of the triple product is distributed randomly to (0, 2 p] (where P is an integer). As a result, the size of the sum of the triple product (bispectrum) becomes close to zero. However, a bias occurs in the phase of the triple product having some relation between these three phases, and as a result, the bispectrum has a value other than 0. Bispectral analysis is to investigate phase coupling in this manner.

In the SynchFastSlow (SFS) calculation step S180, SynchFastSlow (relative synchrony of fast and slow wave, SFS) is obtained by the following equation: SynchFastSlow shall be the ratio of the sizes of the bispectrum of the entire frequency region to the high frequency region from the brain wave.

$$\text{SynchFastSlow} = \log [\text{bispectral power } (0.5 - 47.0 \text{ Hz}) / \text{bispectral power } (40.0 - 47.0 \text{ Hz})]$$

Here, the bispectral power (0.5 - 47.0 Hz) is the bispectrum calculated in the range of 0.5 Hz to 47.0 Hz, and the bispectral power (40.0 - 47.0 Hz) is the calculated bispectrum in the range of 40.0 Hz to 47.0 Hz.

According to circumstances, SynchFastSlow can also be obtained as the log ratio of the bispectrum peak sum in the range of 40.0 Hz and 47.0 Hz (i.e., 40 - 47 Hz region) to the bispectrum peak sum in the range of 0.5 Hz and 47.0 Hz (i.e., 0.5 - 47 Hz region).

In a brain activity determination step, BSR calculated in the BSR calculation step 140, the beta ratio obtained in the beta ratio calculation step S160, and the SynchFastSlow(SFS) calculated in the SynchFastSlow(SFS) step are used to detect brain activity.

Each score is assigned based on BSR, beta ratio, and SynchFastSlow (SFS) for brain activity, and brain activity can be obtained by adding these scores, or by taking an average value of these scores. For example, BSR score is assigned according to BSR score assigned values pre-stored on memory based on the value of BSR, beta ratio is also assigned with a beta ratio score based the beta ratio score assignment on pre-stored on memory, and SynchFastSlow is also assigned a score of SynchFastSlow based on the SynchFastSlow assignment values pre-stored on memory.

According to circumstances, brain activity is each assigned with a score according to BSR, beta ratio, SynchFastSlow (SFS), and QUAZI, respectively, and can be determined as a sum of these scores or taking an average value of these scores.

Alternatively, any one among BSR, beta ratio, and SynchFastSlow (SFS) can be used as brain activity.

The brain activity obtained in this way is used as a CPR signal in the conventional auto cardiopulmonary resuscitation device, and this CPR signal, that is, brain activity signal, is displayed on the monitor unit 395.

In addition, the calculation processing unit determines the degree of cardiopulmonary resuscitation by CPR signals, that is, brain activity signals, and generates motor control signals for changing chest compression rates according the degree of cardiopulmonary resuscitation, and sends them to the chest compression adjusting unit 370. In addition, the calculation processing unit 350 determines the degree of cardiopulmonary resuscitation by using the detected CPR signal, that is, brain activity, and when the degree of cardiopulmonary resuscitation falls outside a predetermined reference range, it generates a defibrillator control signal and sends it to the chest compression adjusting unit 370.

In other words, the BSR, beta ratio, SynchFastSlow (SFS), etc. can be regarded as brain activation indexes, scores are assigned according to each of these brain activation indexes, and brain activity is calculated by adding these scores or taking an average value of these scores, and according to circumstances, any one of BSR, beta ratio, and SynchFastSlow (SFS) can be used as brain activity.

As described above, although the present invention has been described by way of limited examples and drawings, the present invention is not limited to the above-mentioned embodiments, and various modifications and variations are possible from the disclosure for a person of ordinary skill in the art to which the present invention belongs. Therefore, the present invention should be understood only by the scope of the claims set forth below.

[INDUSTRIAL APPLICABILITY]

The auto cardiopulmonary resuscitation device according to the present invention measures electrocardiogram and oxygen saturation during a cardiopulmonary resuscitation procedure, detects brain activity in real time which is a degree of blood circulation to the brain by measuring electroencephalogram, is equipped with a defibrillation function, uses a brain activation index as a CPR signal indicating a degree of cardiopulmonary resuscitation, and is capable of *in vivo* signal remote monitoring in a pre-hospital step. Therefore, the cardiopulmonary resuscitation device can be placed in government offices, schools, family homes, ambulances, etc., to provide appropriate treatments to patients in less time so that the rate of resuscitation of patients can be increased.

**Claims**

1.  A cardiopulmonary resuscitation device (10) configured to use a brain activation index as a CPR signal indicating a degree of cardiopulmonary resuscitation, comprising:

    an electroencephalogram detection unit (330) configured to detect an electroencephalogram signal in the frontal lobe;
    a calculation processing unit (350) configured to detect a brain activation index from the electroencephalogram signal received from the electroencephalogram
    detection unit (330), wherein the calculation processing unit (350) is configured to calculate one
    or more of a burst suppression ratio (BSR), a beta ratio, and a relative synchrony of fast and slow wave (SynchFastSlow, SFS) as the brain activation index;
    a main body portion (100) equipped with a chest compression unit (130) configured to pressure a chest of a patient; and
    a holder (200) configured to support the main body portion (100) and be able to attach or detach the main body portion (100), wherein the holder (200) further comprises:

    a main body holding unit (210) which is U-shaped and bound to an edge of the main body portion (100);
    a holder leg portion (220) which is connected to the main body holding unit (210) to support the main body portion (100); and
    a back support portion (250), wherein one end thereof is connected to the holder leg portion (220) as a means of supporting a back of a patient, wherein the back support portion (250) is composed of two separate boards, and wherein a distance between the two boards can be adjusted to correspond to the width of the body of a patient.

2.  The cardiopulmonary resuscitation device (10) according to claim 1, wherein the calculation processing unit (350) is configured to remove a noise in a electroencephalogram signal received from the electroencephalogram detection unit (330), pass the electroencephalogram signal in which the noise is removed through a 1 Hz low pass filter, perform correction by excluding the signal that passed through the 1Hz low pass filter in the electroencephalogram signal in which the noise has been removed, take a first derivative of the corrected electroencephalogram signal, detect a section in which an amplitude is 5 $\mu$V or less for 0.5 sec or more as a suppression (flat brain wave), and thereby detect BSR.

3. The cardiopulmonary resuscitation device (10) according to claim 1, wherein the calculation processing unit (350) is configured to remove a noise in an electroencephalogram signal received from the electroencephalogram detection unit (330) and calculate a beta ratio from the electroencephalogram signal in which the noise has been removed according to:

Beta Ratio = log [spectral power (30-47 Hz) / spectral power (11-20 Hz)]

(where spectral power (30-47 Hz) is a power spectrum at a frequency of 30 to 47 Hz in electroencephalogram, and spectral power (11-20 Hz) is a power spectrum at a frequency of 11 to 20 Hz).

4. The cardiopulmonary resuscitation device (10) according to claim 1, wherein the calculation processing unit (350) is configured to remove a noise in an electroencephalogram signal received by the electroencephalogram detection unit (330) and calculate SynchFastSlow (SFS) from the electroencephalogram signal in which the noise has been removed according to:

SynchFastSlow=log [bispectral power (0.5-47.0 Hz)/ bispectral power (40.0-47.0 Hz)]

(where bispectral power (0.5-47.0 Hz) is a bispectrum calculated in a range of 0.5 Hz to 47.0 Hz, and bispectral power (40.0-47.0 Hz) is a bispectrum calculated in a range of 40.0 Hz to 47.0 Hz).

5. The cardiopulmonary resuscitation device (10) according to any one of claims 1 to 4, wherein the calculation processing unit (350) is configured to assign a score according to pre-stored distribution values for each of BSR, beta ratio, and SynchFastSlow (SFS) and add up assigned scores of BSR, beta ratio, and SynchFastSlow (SFS) to calculate a brain activity.

6. The cardiopulmonary resuscitation device (10) according to any one of claims 1 to 5, further comprising an angle adjusting unit (230) equipped with a hinge between the holder leg portion (220) and back support portion (250), wherein the angle adjusting unit (230) can be fixed by a stopper.

7. The cardiopulmonary resuscitation device (10) according to claim 6, wherein the angle adjusting unit (230) is configured so that the distance between a base of the main body portion (100) and the back support portion (250) can be adjusted, and wherein the holder leg portion (220) includes two holder legs and is configured so that the patient's neck and head can be positioned between the two holder legs.

8. The cardiopulmonary resuscitation device (10) according to claim 7, wherein the main body portion (100) mounted on the holder (200) is located a predetermined distance above the back support portion (250), and wherein the holder leg is connected at each end of the main body holding unit (210).

9. The cardiopulmonary resuscitation device (10) according to any one of claims 1 to 5, further comprising a wrist fastening unit (214) configured to fasten and secure a wrist of a patient to left and right sides of the main body holding unit (210).

10. The cardiopulmonary resuscitation device (10) according to claim 9, wherein the wrist fastening unit (214) comprises any one of an electrocardiogram sensor and oxygen saturation sensor to detect an electrocardiogram signal or oxygen saturation signal to send to the calculation processing unit (350).

11. The cardiopulmonary resuscitation device (10) according to any one of claims 1 to 5, wherein the back support portion (250) comprises an electrocardiogram sensor to detect an electrocardiogram signal to send to the calculation processing unit (350).

12. The cardiopulmonary resuscitation device (10) according to claim 1, wherein the electroencephalogram detection unit (330) comprises two or four electroencephalogram signal electrodes (331, 332, 337, 338).

13. The cardiopulmonary resuscitation device (10) according to claim 12, wherein the electroencephalogram detection unit (330) is headband-formed.

**14.** The cardiopulmonary resuscitation device (10) according to claim 1, the device further comprising a monitor unit (395) configured to output a brain activation index.

**Patentansprüche**

**1.** Herz-Lungen-Reanimationsvorrichtung (10), die so konfiguriert ist, dass sie einen Gehirnaktivierungsindex als ein CPR-Signal verwendet, das einen Grad der Herz-Lungen-Reanimation anzeigt, umfassend:

eine Elektroenzephalogramm-Erfassungseinheit (330), die so konfiguriert ist, dass sie ein Elektroenzephalogramm-Signal im Frontallappen erfasst;
eine Berechnungsverarbeitungseinheit (350), die so konfiguriert ist, dass sie einen Hirnaktivierungsindex aus dem von der Elektroenzephalogramm-Erfassungseinheit (330) empfangenen Elektroenzephalogramm-Signal erfasst, wobei die Berechnungsverarbeitungseinheit (350) so konfiguriert ist, dass sie ein oder mehrere von einem Burst-Suppression-Verhältnis (BSR), einem Beta-Verhältnis und einer relativen Synchronität von schnellen und langsamen Wellen (SynchFastSlow, SFS) als den Hirnaktivierungsindex berechnet;
einen Hauptkörperabschnitt (100), der mit einer Brustkorbkompressionseinheit (130) ausgestattet ist, die so konfiguriert ist, dass sie einen Druck auf den Brustkorb eines Patienten ausübt; und
einen Halter (200), der so konfiguriert ist, dass er den Hauptkörperabschnitt (100) stützt und in der Lage ist, den Hauptkörperabschnitt (100) zu befestigen oder zu lösen, wobei der Halter (200) ferner umfasst:

eine Hauptkörperhalteeinheit (210), die U-förmig ist und mit einer Kante des Hauptkörperabschnitts (100) verbunden ist;
einen Halterbeinabschnitt (220), der mit der Hauptkörperhalteeinheit (210) verbunden ist, um den Hauptkörperabschnitt (100) zu stützen; und
einen Rückenstützabschnitt (250), wobei ein Ende davon mit dem Halterbeinabschnitt (220) als ein Mittel zum Stützen eines Rückens eines Patienten verbunden ist, wobei der Rückenstützabschnitt (250) aus zwei separaten Platten zusammengesetzt ist, und wobei ein Abstand zwischen den zwei Platten eingestellt werden kann, um der Breite des Körpers eines Patienten zu entsprechen.

**2.** Herz-Lungen-Reanimationsvorrichtung (10) nach Anspruch 1, wobei die Berechnungsverarbeitungseinheit (350) so konfiguriert ist, dass sie ein Rauschen in einem Elektroenzephalogramm-Signal, das von der Elektroenzephalogramm-Erfassungseinheit (330) empfangen wird, entfernt, das Elektroenzephalogramm-Signal, in dem das Rauschen entfernt wurde, durch einen 1-Hz-Tiefpassfilter leitet, eine Korrektur durch Ausschließen des Signals, das den 1 Hz-Tiefpassfilter durchlaufen hat, in dem Elektroenzephalogramm-Signal, in dem das Rauschen entfernt wurde, durchführt, eine erste Ableitung des korrigierten Elektroenzephalogramm-Signals nimmt, einen Abschnitt, in dem eine Amplitude 5 μV oder weniger für 0,5 Sekunden oder länger beträgt, als Supression (flache Hirnwelle) erfasst und dadurch BSR erfasst.

**3.** Herz-Lungen-Reanimationsvorrichtung (10) nach Anspruch 1, wobei die Berechnungsverarbeitungseinheit (350) so konfiguriert ist, dass sie ein Rauschen in einem Elektroenzephalogramm-Signal, das von der Elektroenzephalogramm-Erfassungseinheit (330) empfangen wird, entfernt und ein Beta-Verhältnis aus dem Elektroenzephalogramm-Signal, in dem das Rauschen entfernt wurde, gemäß Folgendem berechnet:

$$\text{Beta-Verhältnis} = \log[\text{Spektralleistung (30-47 Hz)} / \text{Spektralleistung (11-20 Hz)}]$$

(wobei die Spektralleistung (30-47 Hz) ein Leistungsspektrum bei einer Frequenz von 30 bis 47 Hz im Elektroenzephalogramm und die Spektralleistung (11-20 Hz) ein Leistungsspektrum bei einer Frequenz von 11 bis 20 Hz ist).

**4.** Herz-Lungen-Reanimationsvorrichtung (10) nach Anspruch 1, wobei die Berechnungsverarbeitungseinheit (350) so konfiguriert ist, dass sie ein Rauschen in einem Elektroenzephalogramm-Signal, das von der Elektroenzephalogramm-Erfassungseinheit (330) empfangen wird, entfernt und SynchFastSlow (SFS) aus dem Elektroenzephalogramm-Signal, in dem das Rauschen entfernt wurde, gemäß Folgendem berechnet:

$$\text{SynchFastSlow} = \log[\text{Bispektralleistung (0,5-47,0 Hz)} / \text{Bispektralleistung (40,0-47,0 Hz)}]$$

(wobei die Bispektralleistung (0,5-47,0 Hz) ein in einem Bereich von 0,5 Hz bis 47,0 Hz berechnetes Bispektrum ist und die Bispektralleistung (40,0-47,0 Hz) ein in einem Bereich von 40,0 Hz bis 47,0 Hz berechnetes Bispektrum ist).

5. Herz-Lungen-Reanimationsvorrichtung (10) nach einem der Ansprüche 1 bis 4, wobei die Berechnungsverarbeitungseinheit (350) so konfiguriert ist, dass sie eine Punktzahl gemäß vorgespeicherten Verteilungswerten für jede BSR, jedes Beta-Verhältnis und jede SynchFastSlow (SFS) zuweist und zugewiesene Punktzahlen von BSR, Beta-Verhältnis und SynchFastSlow (SFS) addiert, um eine Gehirnaktivität zu berechnen.

6. Herz-Lungen-Reanimationsvorrichtung (10) nach einem der Ansprüche 1 bis 5, ferner umfassend eine Winkeleinstelleinheit (230), die mit einem Scharnier zwischen dem Halterbeinabschnitt (220) und dem Rückenstützabschnitt (250) ausgestattet ist,
wobei die Winkelverstelleinheit (230) durch einen Stopper fixiert werden kann.

7. Herz-Lungen-Reanimationsvorrichtung (10) nach Anspruch 6, wobei die Winkeleinstelleinheit (230) so konfiguriert ist, dass der Abstand zwischen einer Basis des Hauptkörperabschnitts (100) und dem Rückenstützabschnitt (250) eingestellt werden kann, und wobei der Halterbeinabschnitt (220) zwei Halterbeine umfasst und so konfiguriert ist, dass der Hals und der Kopf des Patienten zwischen den beiden Halterbeinen positioniert werden können.

8. Herz-Lungen-Reanimationsvorrichtung (10) nach Anspruch 7, wobei der am Halter (200) angebrachte Hauptkörperabschnitt (100) in einem vorbestimmten Abstand über dem Rückenstützabschnitt (250) angeordnet ist und wobei das Halterbein an jedem Ende der Hauptkörperhalteeinheit (210) verbunden ist.

9. Herz-Lungen-Reanimationsvorrichtung (10) nach einem der Ansprüche 1 bis 5, die ferner eine Handgelenkbefestigungseinheit (214) umfasst, die so konfiguriert ist, dass sie ein Handgelenk eines Patienten an der linken und rechten Seite der Hauptkörperhalteeinheit (210) befestigt und sichert.

10. Herz-Lungen-Reanimationsvorrichtung (10) nach Anspruch 9, wobei die Handgelenkbefestigungseinheit (214) einen von einem Elektrokardiogrammsensor und einem Sauerstoffsättigungssensor umfasst, um ein Elektrokardiogramm-Signal oder ein Sauerstoffsättigungssignal zu erfassen und an die Berechnungsverarbeitungseinheit (350) zu senden.

11. Herz-Lungen-Reanimationsvorrichtung (10) nach einem der Ansprüche 1 bis 5, wobei der Rückenstützenabschnitt (250) einen Elektrokardiogrammsensor zur Erfassung eines Elektrokardiogramm-Signals umfasst, das an die Berechnungsverarbeitungseinheit (350) gesendet wird.

12. Herz-Lungen-Reanimationsvorrichtung (10) nach Anspruch 1, wobei die Elektroenzephalogramm-Erfassungseinheit (330) zwei oder vier Elektroenzephalogramm-Signalelektroden (331, 332, 337, 338) umfasst.

13. Herz-Lungen-Reanimationsvorrichtung (10) nach Anspruch 12, wobei die Elektroenzephalogramm-Erfassungseinheit (330) als Kopfband ausgebildet ist.

14. Herz-Lungen-Reanimationsvorrichtung (10) nach Anspruch 1, wobei die Vorrichtung ferner eine Überwachungseinheit (395) umfasst, die so konfiguriert ist, dass sie einen Gehirnaktivierungsindex ausgibt.

**Revendications**

1. Dispositif de réanimation cardio-pulmonaire (10) configuré pour utiliser un indice d'activation cérébrale comme signal de réanimation cardio-pulmonaire indiquant un degré de réanimation cardio-pulmonaire, comprenant :

une unité de détection d'électroencéphalogramme (330) configurée pour détecter un signal d'électroencéphalogramme dans le lobe frontal ;
une unité de traitement des calculs (350) configurée pour détecter un indice d'activation cérébrale à partir du signal d'électroencéphalogramme reçu de l'unité de détection d'électroencéphalogramme (330), l'unité de traitement des calculs (350) étant configurée pour calculer un ou plusieurs indices parmi un rapport de suppression des salves (BSR), un rapport bêta et une synchronisation relative des ondes rapides et lentes (SynchFastSlow, SFS) en tant qu'indice d'activation cérébrale ;
une partie de corps principal (100) équipée d'une unité de compression thoracique (130) configurée pour presser la poitrine d'un patient ; et
un support (200) configuré pour soutenir la partie de corps principal (100) et pouvoir attacher ou détacher la partie de corps principal (100), dans lequel le support (200) comprend en outre :

une unité de maintien de corps principal (210) qui est en forme de U et liée à un bord de la partie de corps principal (100) ;
une partie de jambe de support (220) qui est reliée à l'unité de support de corps principal (210) pour soutenir la partie de corps principal (100) ; et
une partie de support dorsal (250), dont une extrémité est reliée à la partie de jambe de support (220) pour soutenir le dos d'un patient, dans laquelle la partie de support dorsal (250) est composée de deux planches séparées, et dans laquelle une distance entre les deux planches peut être ajustée pour correspondre à la largeur du corps d'un patient.

2. Dispositif de réanimation cardio-pulmonaire (10) selon la revendication 1, dans lequel l'unité de traitement des calculs (350) est configurée pour supprimer un bruit dans un signal d'électroencéphalogramme reçu depuis l'unité de détection d'électroencéphalogramme (330), faire passer le signal d'électroencéphalogramme dans lequel le bruit a été supprimé à travers un filtre passe-bas de 1 Hz, effectuer une correction en excluant le signal qui a traversé le filtre passe-bas de 1 Hz dans le signal d'électroencéphalogramme dans lequel le bruit a été supprimé, prendre une dérivée première du signal d'électroencéphalogramme corrigé, détecter une section dans laquelle une amplitude est de 5 $\mu$V ou moins pendant 0.5 secondes ou plus comme une suppression (onde cérébrale plate), et détecter ainsi le BSR.

3. Dispositif de réanimation cardio-pulmonaire (10) selon la revendication 1, dans lequel l'unité de traitement des calculs (350) est configurée pour supprimer un bruit dans un signal d'électroencéphalogramme reçu depuis l'unité de détection d'électroencéphalogramme (330) et pour calculer un rapport bêta à partir du signal d'électroencéphalogramme dans lequel le bruit a été supprimé selon ce qui suit :

Rapport bêta = log [puissance spectrale (30-47 Hz) / puissance spectrale (11-20 Hz)].

(où la puissance spectrale (30-47 Hz) est un spectre de puissance à une fréquence de 30 à 47 Hz dans l'électroencéphalogramme, et la puissance spectrale (11-20 Hz) est un spectre de puissance à une fréquence de 11 à 20 Hz).

4. Dispositif de réanimation cardio-pulmonaire (10) selon la revendication 1, dans lequel l'unité de traitement des calculs (350) est configurée pour supprimer un bruit dans un signal d'électroencéphalogramme reçu par l'unité de détection d'électroencéphalogramme (330) et calculer SynchFastSlow (SFS) à partir du signal d'électroencéphalogramme dans lequel le bruit a été supprimé selon ce qui suit :

SynchFastSlow=log [puissance bispectrale (0,5-47,0 Hz)/ puissance bispectrale (40,0-47,0 Hz)].

(où la puissance bispectrale (0,5-47,0 Hz) est un bispectre calculé dans une plage de 0,5 Hz à 47,0 Hz, et la puissance bispectrale (40,0-47,0 Hz) est un bispectre calculé dans une plage de 40,0 Hz à 47,0 Hz).

5. Dispositif de réanimation cardio-pulmonaire (10) selon l'une des revendications 1 à 4, dans lequel l'unité de traitement des calculs (350) est configurée pour attribuer un score en fonction de valeurs de distribution préenregistrées pour chaque BSR, du rapport bêta et de SynchFastSlow (SFS) et pour additionner les scores attribués de BSR, du rapport bêta et de SynchFastSlow (SFS) afin de calculer une activité cérébrale.

6. Dispositif de réanimation cardio-pulmonaire (10) selon l'une des revendications 1 à 5, comprenant en outre une unité de réglage d'angle (230) équipée d'une charnière entre la partie de jambe de support (220) et la partie de support dorsal (250),
dans lequel l'unité de réglage d'angle (230) peut être fixée par un bouchon.

7. Dispositif de réanimation cardio-pulmonaire (10) selon la revendication 6, dans lequel l'unité de réglage d'angle (230) est configurée de manière à ce que la distance entre une base de la partie de corps principal (100) et la partie de support dorsal (250 ) puisse être réglée, et dans lequel la partie de jambe de support (220) comprend deux jambes de support et est configurée de manière à ce que le cou et la tête du patient puissent être positionnés entre les deux jambes de support.

8. Dispositif de réanimation cardio-pulmonaire (10) selon la revendication 7, dans lequel la partie de corps principal (100) montée sur le support (200) est située à une distance prédéterminée au-dessus de la partie de support dorsal (250), et dans lequel la jambe de support est reliée à chaque extrémité de l'unité de maintien de corps principal (210).

9. Dispositif de réanimation cardio-pulmonaire (10) selon l'une des revendications 1 à 5, comprenant en outre une unité de fixation de poignet (214) configurée pour fixer et sécuriser un poignet d'un patient sur les côtés gauche et droit de l'unité de maintien de corps principal (210).

10. Dispositif de réanimation cardio-pulmonaire (10) selon la revendication 9, dans lequel l'unité de fixation de poignet (214) comprend l'un d'un capteur d'électrocardiogramme et d'un capteur de saturation en oxygène pour détecter un signal d'électrocardiogramme ou un signal de saturation en oxygène à envoyer à l'unité de traitement des calculs (350).

11. Dispositif de réanimation cardio-pulmonaire (10) selon l'une quelconque des revendications 1 à 5, dans lequel la partie de support dorsal (250) comprend un capteur d'électrocardiogramme pour détecter un signal d'électrocardiogramme à envoyer à l'unité de traitement de calcul (350).

12. Dispositif de réanimation cardio-pulmonaire (10) selon la revendication 1, dans lequel l'unité de détection d'électroencéphalogramme (330) comprend deux ou quatre électrodes de signal d'électroencéphalogramme (331, 332, 337, 338).

13. Dispositif de réanimation cardio-pulmonaire (10) selon la revendication 12, dans lequel l'unité de détection d'électroencéphalogramme (330) est formée d'un bandeau.

14. Dispositif de réanimation cardio-pulmonaire (10) selon la revendication 1, le dispositif comprenant en outre une unité de surveillance (395) configurée pour émettre un indice d'activation cérébrale.

【Figure 1】

【Figure 2】

【Figure 3】

【Figure 4】

| 310 Electrocardiogram detection unit | 340 A/D conversion unit | 350 Calculation processing unit | 370 Chest compression adjusting unit |
|---|---|---|---|

【Figure 5】

【Figure 6】

【Figure 7】

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020110014186

- US 20140324763 A1